# FASCICULE DE BREVET EUROPEEN

(11) **EP 1 496 961 B1**
(45) Date de publication et mention de la délivrance du brevet: **09.11.2005**
(21) Numéro de dépôt: 03746847.7
(22) Date de dépôt: 18.04.2003
(51) Int. Cl.: A61M 5/00

(54) **EMBALLAGE POUR OBJETS STERILES OU A STERILISER**
VERPACKUNG FÜR STERILE GEGENSTÄNDE ODER ZU STERILISIERENDE GEGENSTÄNDE
PACKAGING FOR STERILE OBJECTS OR OBJECTS TO BE STERILIZED

(30) Priorité: 22.04.2002 FR 0204996
(43) Date de publication de la demande: 19.01.2005
(73) Titulaire: Becton Dickinson France, 38800 Le Pont de Claix (FR)
(72) Inventeur: RAYNAL-OLIVE, Claire, 38450 Vif (FR); GRIMARD, Jean-Pierre, 38450 Vif (FR)
(74) Mandataire: Brédeville, Odile Marie
(86) Numéro de dépôt international: PCT/FR2003/001260
(87) Numéro de publication internationale: WO 2003/089028

(56) Documents cités:
- WO-A-01/82817
- US-A- 4 402 407
- US-B1- 6 263 641

## Description

La présente invention concerne un emballage destiné à être utilisé pour transporter des objets stériles ou à stériliser, un procédé de fabrication de cet emballage, un procédé de stérilisation et de décontamination utilisant cet emballage, et l'utilisation de cet emballage dans un procédé de stérilisation et de décontamination.

L'emballage selon l'invention peut notamment être utilisé pour transporter des composants de seringues, en particulier des corps de seringue destinés à être ultérieurement remplis par un produit actif ou un médicament.

Les conditions de stérilité dans lesquelles doivent se dérouler certaines étapes de manipulation ou de transport d'objets destinés à un usage médical sont très contraignantes, en particulier dans l'industrie pharmaceutique. Il est donc d'une grande importance de réaliser des emballages compatibles avec de telles exigences.

Dans la suite de la description, il sera fait mention d'un matériau sélectivement étanche qu'il convient de définir. Par l'expression "sélectivement étanche" telle qu'utilisée dans la présente description ainsi que dans les revendications, on entend que le matériau est conçu, en termes de structure, de manière à contrôler tout échange de part et d'autre dudit matériau, et ainsi notamment de l'intérieur de l'emballage vers l'environnement extérieur de celui-ci. Ceci signifie entre autres que l'emballage est étanche, individuellement ou en combinaison, à la contamination par des microorganismes, bactéries et/ou un matériau biologiquement actif, susceptibles de venir en contact avec l'emballage lors de sa manipulation, tout .en restant perméable à un gaz de stérilisation par exemple du type ETO (oxyde d'éthylène).

II est connu de placer des objets stériles ou à stériliser dans une boîte en matière plastique, de fixer ensuite une feuille de couverture en matériau sélectivement étanche sur cette boîte de manière à sceller cette dernière, de placer l'emballage ainsi formé dans un deuxième emballage comprenant une fenêtre fermée par une feuille en matériau sélectivement étanche, et de procéder à une stérilisation de l'ensemble par un gaz du type ETO. L'emballage ainsi stérilisé est placé dans une boîte par exemple en carton pour son expédition ; à destination, il est procédé à l'ouverture de la boîte en carton et dudit deuxième emballage, puis à la décontamination et à l'ouverture dudit emballage.

Dans le cas de composants de seringues, il est connu d'utiliser une boîte notamment en polystyrène et une feuille de couverture en matériau commercialisé sous la marque TYVEK® par la société Du Pont de Nemours. Ce matériau est formé à base de filaments de PEHD (polyéthylène haute densité), liés notamment par l'intermédiaire de chaleur et de pression.

Le document US 6, 263, 641 décrit un tel emballage comprenant en outre une couche d'un matériau pouvant être du polyéthylène, de la mousse ou du plastique, cette couche reposant sur les objets contenus dans l'emballage.

Pour ledit deuxième emballage, il est connu d'utiliser un sac en matière plastique, la feuille de fermeture de la fenêtre que comprend ce sac étant également en "TYVEK®".

A destination, après retrait de ce deuxième emballage, la boîte est exposée à un gaz de décontamination, par exemple à des vapeurs de peroxyde d'hydrogène, afin de réaliser sa décontamination. Cette exposition se fait dans un sas ou un tunnel d'acheminement de cette boîte à une zone stérile.

Ce type de décontamination est bien adapté à certaines utilisations, notamment à la décontamination d'emballages contenant des corps de seringues tels que précités. La demanderesse a toutefois pu constater que dans certains cas, il existait des interactions indésirables entre les objets contenus dans l'emballage, en particulier des corps de seringue, et les produits avec lesquels ces objets sont ensuite en contact, en particulier des produits actifs ou des médicaments venant ultérieurement remplir les corps de seringue.

L'invention vise à remédier à cet inconvénient important. Son objectif est donc de fournir un emballage pour des objets stériles ou à stériliser, pouvant être stérilisé au moyen d'un gaz de stérilisation par exemple du type ETO (oxyde d'éthylène), et pouvant être décontaminé au moyen d'un gaz de décontamination, par exemple au moyen de vapeurs de peroxyde d'hydrogène, sans qu'il existe ultérieurement des interactions indésirables entre les objets contenus dans l'emballage, en particulier des corps de seringue, et les produits avec lesquels ces objets sont destinés à être en contact, en particulier des produits actifs ou des médicaments venant ultérieurement remplir des corps de seringue.

En d'autres termes, l'invention vise à fournir un emballage efficace à l'égard d'éventuelles pénétrations du gaz de décontamination, par exemple des vapeurs de peroxyde d'hydrogène, lors du processus de décontamination sans amoindrir significativement l'aptitude de cet emballage à être stérilisé au moyen d'un gaz de stérilisation.

L'objectif de la présente invention est également de fournir un procédé pour la réalisation de cet emballage.

Un autre objectif de la présente invention est de fournir un procédé amélioré de stérilisation et de décontamination, utilisant ledit emballage.

L'emballage concerné comprend, de manière connue en soi, une boîte destinée à recevoir les objets stériles ou à stériliser et une feuille de couverture en matériau sélectivement étanche, fixée sur la boîte de manière à sceller cette dernière de façon étanche.

Selon l'invention, l'emballage comprend au moins une couche d'un matériau formant un écran au moins partiel vis à vis d'un gaz de décontamination, par exemple vis-à-vis de vapeurs de peroxyde d'hydrogène, et/ou à même d'absorber un gaz de décontamination, par exemple des vapeurs de peroxyde d'hydrogène, cette couche ayant une forme et des dimensions telles qu'elle puisse être placée dans la boîte le long de la feuille de couverture et qu'elle s'étende, dans cette position, au-dessus des objets contenus dans l'emballage, la ou lesdites couches ou les objets étant mobiles entre une position de diffusion, permettant une diffusion non restreinte du gaz de stérilisation sur, entre et éventuellement dans, les objets, et une position de non diffusion, permettant une diffusion restreinte, voire empêchée, du gaz de décontamination, par exemple des vapeurs de peroxyde d'hydrogène, sur, entre et éventuellement dans, ces mêmes objets, et étant caractérisé en ce qu'il comprend en outre une plaque ou une grille pourvue de saillies, conformée pour, dans ladite position de diffusion, permettre la diffusion non restreinte du gaz de stérilisation sur, entre et éventuellement dans, les objets, et, dans ladite position de non diffusion, restreindre ou interdire la diffusion du gaz de décontamination, par exemple des vapeurs de peroxyde d'hydrogène, sur, entre et éventuellement dans, ces mêmes objets.

Le procédé selon l'invention de fabrication de cet emballage comprend les étapes consistant à :
- utiliser au moins un matériau à même de former un écran au moins partiel vis à vis d'un gaz de décontamination, par exemple vis-à-vis de vapeurs de peroxyde d'hydrogène, et/ou à même d'absorber un gaz de décontamination, par exemple des vapeurs de peroxyde d'hydrogène,
- aménager au moins une couche de ce matériau, en choisissant la forme et les dimensions de cette couche de telle sorte que celle-ci puisse être placée dans la boîte le long de la feuille de couverture et qu'elle s'étende, dans cette position, au-dessus des objets contenus dans l'émballage, et présentant l'étape caractérisante de
- aménager une plaque ou une grille pourvue de saillies, conformée pour, dans une position de diffusion, permettre la diffusion non restreinte du gaz de stérilisation sur, entre et éventuellement dans, les objets, et, dans une position de non diffusion, restreindre ou interdire la diffusion du gaz de décontamination, par exemple des vapeurs de peroxyde d'hydrogène, sur, entre et éventuellement dans, ces mêmes objets.

L'invention concerne également l'utilisation de l'emballage précité dans un procédé de décontamination de cet emballage par un gaz de décontamination, par exemple par des vapeurs de peroxyde d'hydrogène.

La demanderesse a en effet pu constater que des résidus de peroxyde d'hydrogène se retrouvaient sur les objets contenus dans la boîte lorsque la feuille en matériau sélectivement étanche utilisée pour sa perméabilité au gaz de stérilisation n'est pas suffisamment étanche aux vapeurs de peroxyde d'hydrogène, comme cela s'avère être parfois le cas du "TYVEK"® utilisé seul, en tant que feuille de couverture, et que ces résidus étaient à l'origine des interactions indésirables précitées. Ces interactions se produisent d'autant plus dans le cas de corps de seringue que lesdits résidus s'accumulent dans ces corps de seringue du fait que les vapeurs de peroxyde d'hydrogène sont plus lourdes que l'air contenu dans l'emballage.

L'invention solutionne ce problème, sinon totalement, tout au moins en grande partie, en prévoyant au moins une couche formant un écran au moins partiel vis à vis d'un gaz de décontamination, par exemple vis-à-vis de vapeurs de peroxyde d'hydrogène, et/ou à même d'absorber un gaz de décontamination, par exemple les vapeurs de peroxyde d'hydrogène, qui pourrait rester dans la boîte à la fin du processus de décontamination et au moins une plaque ou une grille pourvue de saillies, conformée pour, dans une position de diffusion, permettre la diffusion non restreinte du gaz de stérilisation sur, entre et éventuellement dans, les objets, et, dans une position de non diffusion, restreindre ou interdire la diffusion du gaz de décontamination, par exemple des vapeurs de peroxyde d'hydrogène, sur, entre et éventuellement dans, ces mêmes objets.

L'expression "écran au moins partiel" signifie que le rôle de cet écran est de limiter, sinon d'interdire, la pénétration d'un gaz de décontamination, par exemple de vapeurs de peroxyde d'hydrogène, dans l'emballage ou l'échange de ce gaz ou de ces vapeurs entre l'intérieur et l'extérieur de l'emballage.

Ladite couche ou au moins une desdites couches peut être rapportée sur la feuille de couverture notamment par collage ou soudure ; cette ou ces couches sont alors dimensionnées de manière à délimiter sur la feuille de couverture une zone périphérique de fixation de cette feuille de couverture à la boîte.

Ladite couche ou au moins une desdites couches peuvent également être simplement disposées sur les objets placés à l'intérieur de la boîte, préalablement au scellage de la feuille de couverture, ou sur des appuis prévus à cet effet, ou sur une pièce de positionnement des objets, placée dans cette boîte.

L'emballage peut également comprendre au moins une desdites couches rapportée sur la feuille de couverture et au moins une autre desdites couches disposée à l'intérieur de la boîte.

De préférence, l'emballage comprend plusieurs couches de matériau pour former ledit écran.

Les couches peuvent alors être identiques d'une couche à l'autre. Elles forment conjointement ledit écran et/ou permettent conjointement d'obtenir l'absorption recherchée du gaz de décontamination, en particulier des vapeurs de peroxyde d'hydrogène.

Ces mêmes couches peuvent également être différentes. L'emballage peut dans ce cas comprendre une ou plusieurs couches à même de former ledit écran et une ou plusieurs couches à même de réaliser ladite absorption.

Selon une possibilité, la ou les couches à même de former ledit écran sont conformées pour délimiter, lorsqu'elles sont en place dans la boîte, un ou plusieurs interstices ou ouvertures latérales ou périphériques entre leurs bords et les parois de la boîte.

Ces interstices ou ouvertures permettent la diffusion du gaz de stérilisation mais restreignent fortement la possibilité pour le gaz de décontamination, par exemple pour les vapeurs de peroxyde d'hydrogène, de s'introduire dans l'emballage au cours du processus de décontamination. La durée et les conditions opératoires de ce processus de décontamination sont en effet nettement moins contraignantes que celles du processus de stérilisation puisqu'il ne s'agit, dans ce processus de décontamination, que de stériliser la surface extérieure de l'emballage.

Pour ménager des interstices, la ou lesdites couches peuvent présenter des dimensions inférieures à celles de la boîte, de sorte qu'elles délimitent un ou plusieurs interstices entre leurs bords et les parois de cette boîte ; pour ménager des ouvertures, cette ou ces mêmes couches peuvent comprendre, au niveau de leurs bords, des encoches et/ou des découpes en forme de crénaux.

De préférence, l'emballage est conformé de telle sorte que la ou lesdites couches, ou les objets contenus dans l'emballage, soient mobiles entre une position de diffusion, permettant une diffusion non restreinte du gaz de stérilisation sur, entre et éventuellement dans, les objets, et une position de non diffusion, permettant une diffusion restreinte, voire empêchée, du gaz de décontamination, par exemple des vapeurs de peroxyde d'hydrogène, sur, entre et éventuellement dans, ces mêmes objets.

Le procédé de stérilisation et de décontamination selon l'invention comprend alors les étapes consistant à :
- placer l'emballage en position de diffusion au cours du processus de stérilisation ; et
- placer l'emballage en position de non diffusion au cours du processus de décontamination.

Selon une forme de réalisation possible de l'invention dans ce cas, l'emballage est conformé de telle sorte que la ou lesdites couches et/ou les objets passent de la position de diffusion à la position de non diffusion par gravité selon que l'emballage est placé dans une première position, correspondant à la position de diffusion, ou qu'il est placé dans une deuxième position, renversée par rapport à ladite première position, correspondant à position de non diffusion.

Le procédé de stérilisation et de décontamination selon l'invention comprend alors les étapes consistant à :
- placer l'emballage dans une première position au cours du processus de stérilisation, dans laquelle la ou lesdites couches et les objets sont en position de diffusion, de sorte que cette ou ces couches ne restreignent que modérement, voire pas du tout, la diffusion du gaz de stérilisation sur, entre et éventuellement dans, les objets ; et
- placer l'emballage dans une deuxième position au cours du processus de décontamination, dans laquelle la ou lesdites couches et les objets sont en position de non diffusion, de sorte que cette ou ces couches restreignent, voire interdisent, la diffusion du gaz de décontamination, par exemple des vapeurs de peroxyde d'hydrogène, sur, entre et éventuellement dans, ces objets.

La ou lesdites couches peuvent être simplement mobiles entre une position de non contact des objets, correspondant à ladite position de diffusion, et une position de contact des objets, correspondant à ladite position de non diffusion.

L'emballage comprend une plaque ou une grille pourvue de saillies, conformée pour, dans une position par rapport aux objets, permettre ladite diffusion et, dans une autre position par rapport aux objets, restreindre ou interdire cette diffusion.

Selon une forme de réalisation de l'invention, l'emballage comprend deux couches du matériau commercialisé sous la référence "TYVEK® 1073 B" par la société Du Pont de Nemours.

Selon une autre forme de réalisation de l'invention, l'emballage comprend deux couches de papier grade médical, notamment en matériau commercialisé sous les dénominations "STERISHEET®", "PROPYPEL®", "ETHYPEL®", "ARPEEL®", "TALTER®" ou "TRANSPEL®" par la société Arjo Wiggins.

Selon encore une autre forme de réalisation de l'invention, l'emballage comprend deux couches dont une est en matériau commercialisé sous la référence "TYVEK® 1073 B" par la société Du Pont de Nemours et l'autre est en papier grade médical, notamment en matériau commercialisé sous les dénominations "STERISHEET®", "PROPYPEL®", "ETHYPEL®", "ARPEEL®", "TALTER®" ou "TRANSPEL®" par la société Arjo Wiggins.

Selon encore une autre forme de réalisation de l'invention, l'emballage comprend deux couches dont une est en matériau commercialisé sous la référence "TYVEK® 1073 B" par la société Du Pont de Nemours et l'autre est en matériau commercialisé sous la référence "TYVEK® 2FS" par la société Du Pont de Nemours.

Selon encore une autre forme de réalisation de l'invention, l'emballage comprend deux couches dont une est en matériau commercialisé sous la référence "TYVEK® 2FS" par la société Du Pont de Nemours et l'autre est en papier grade médical, notamment en matériau commercialisé sous les dénominations "STERISHEET®", "PROPYPEL®", "ETHYPEL®", "ARPEEL®", "TALTER®" ou "TRANSPEL®" par la société Arjo Wiggins.

Dans les deux cas d'emploi du "TYVEK® 2FS", le côté lisse de ce matériau est de préférence placé en contact avec les objets contenus dans l'emballage de manière à restreindre l'accès des vapeurs de peroxyde d'hydrogène aux objets contenus dans l'emballage, particulièrement aux volumes internes de ces objets comme dans le cas de corps de seringue. Dans ce cas de corps de seringue, les couches de "TYVEK® 2FS" reposent contre les extrémités proximales des corps de seringue, c'est-à-dire celles opposées aux extrémités recevant les aiguilles.

Pour sa bonne compréhension, l'invention est à nouveau décrite ci-dessous en référence au dessin schématique annexé, représentant, à titre d'exemples non limitatifs, plusieurs formes de réalisation possibles de l'emballage qu'elle concerne.
La figure 1 en est une vue en coupe longitudinale selon une première forme de réalisation, dans la position renversée dans laquelle l'emballage est placé au cours d'un processus de stérilisation de cet emballage au moyen d'un gaz de stérilisation, notamment du type ETO (oxyde d'éthylène) ;
la figure 2 en est une vue similaire à la figure 1, dans une position non renversée dans laquelle l'emballage est placé au cours d'un processus ultérieur de décontamination au moyen de vapeurs de peroxyde d'hydrogène ;
la figure 3 est une vue partielle et en coupe longitudinale selon une deuxième forme de réalisation, dans la position renversée dans laquelle l'emballage est placé au cours dudit processus de stérilisation ; et
la figure 4 est une vue similaire à la figure 3, dans une position non renversée dans laquelle l'emballage est placé au cours dudit processus ultérieur de décontamination.

Par simplification, les parties ou éléments de la première forme de réalisation qui se retrouvent de manière identique ou similaire dans la deuxième forme de réalisation seront désignés par les mêmes références numériques et ne seront pas redécrits en détails.

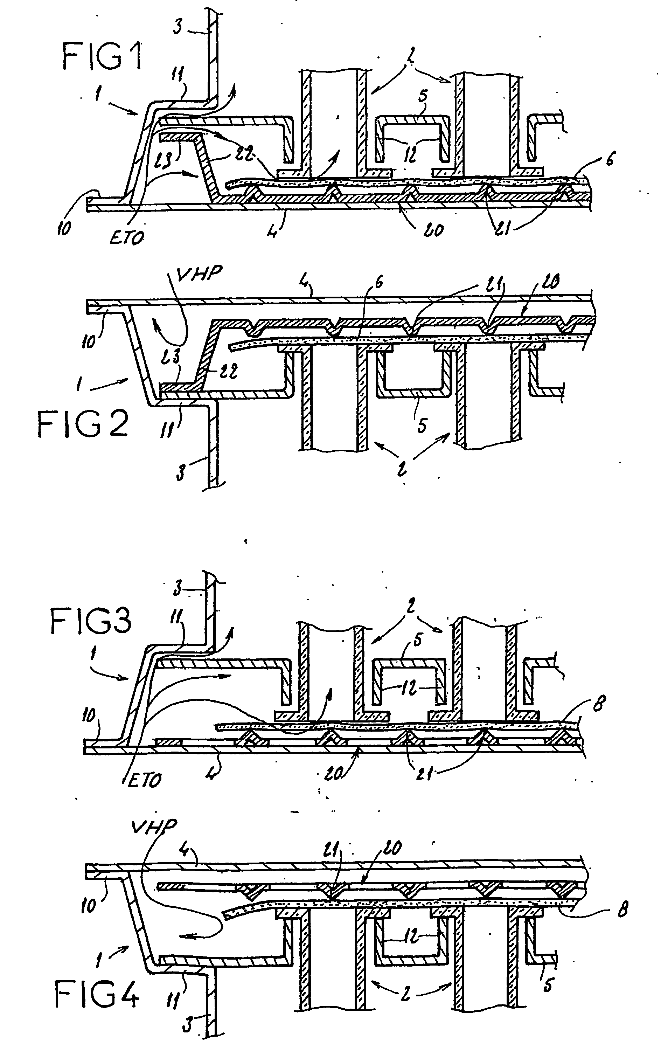

Les figures 1 et 2 représentent un emballage 1 utilisé pour transporter des composants de seringues, en particulier, dans l'exemple représenté, des corps de seringue 2 destinés à être ultérieurement remplis par un produit actif ou un médicament.

L'emballage 1 comprend une boîte 3, une feuille de couverture 4 scellée sur la boîte 3, un plateau 5 non fixé à la boîte 3 et ayant des canons 12 qui autorisent le coulissement libre des corps de seringue 2 en eux, une couche 6 en matériau sélectivement étanche, par exemple en TYVEK® et une plaque 20 située entre la couche 6 et la feuille 4..

La boîte 3 est en polystyrène et comprend une bride périphérique 10 permettant le scellage de la feuille 4. Elle forme également un épaulement supérieur 11 de réception du plateau 5.

La feuille de couverture 4 est en "TYVEK® 1073 B", matériau commercialisé par la société Du Pont de Nemours, formé à base de filaments de PEHD (polyéthylène haute densité) liés notamment par l'intermédiaire de chaleur et de pression. Ce matériau est "sélectivement étanche" en ce sens qu'il est étanche à la contamination de l'intérieur de l'emballage 1 par des microorganismes, bactéries ou autre matériau biologiquement actif, tout en restant perméable à un gaz de stérilisation de l'intérieur de l'emballage 1 par exemple du type ETO (oxyde d'éthylène).

La plaque 20 est thermoformée et présente des saillies 21 sous forme de nervures et/ou de plots. Elle comprend également une jupe latérale 22 terminée par un rebord 23 s'étendant parallèlement au plan de la plaque 20, la hauteur de cette jupe 22 étant notablement inférieure à la distance séparant la face du plateau 5 tournée vers la feuille 4 de la face interne de cette feuille 4.

En pratique, un plateau 5 comportant des corps de seringue 2 est placé dans la boîte 3, la couche 6 est placée par-dessus les corps de seringue. La plaque 20 est placée sur la couche 6, les saillies 21 dirigées vers cette couche 6 et la feuille de couverture est scellée sur la boîte 3. L'emballage 1 ainsi constitué est placé dans un deuxième emballage formé par un sac en matière plastique comprenant une fenêtre fermée par une feuille en « TYVEK® », et l'ensemble est placé dans une boîte en carton ; il est procédé ensuite à une stérilisation de cet ensemble par un gaz du type ETO, l'emballage 1 étant placé dans la position renversée montrée sur la figure 1 au cours du processus de stérilisation.

Après expédition, il est procédé à destination à l'ouverture de la boîte en carton et dudit deuxième emballage, puis à la décontamination de l'emballage 1. Pour ce faire, cet emballage 1 est placé dans la position non renversée montrée sur la figure 2 puis est exposé à des vapeurs de peroxyde d'hydrogène dans un sas ou un tunnel d'acheminement de cet emballage 1 à une zone stérile.

Comme cela se déduit par comparaison des figures 1 et 2, dans la position renversée de l'emballage 1 montrée sur la figure 1, le plateau 5 et la plaque 20 se déplacent par gravité vis-à-vis de la boîte 3 jusqu'à ce que la plaque 20 repose contre la feuille 4. Les corps de seringue 2 portent alors contre la couche 6, elle-même reposant contre les saillies 21. Les corps de seringue 2 ne portent que ponctuellement contre les saillies 21 de sorte que leur volume interne n'est pas isolé du reste du volume interne de l'emballage 1 et que le gaz de stérilisation peut se diffuser sur l'ensemble de ce volume, ainsi que figure la flèche ETO. Dans cette même position, le plateau 5 est écarté de l'épaulement 11 et le rebord 23 est à distance du plateau 5, ce qui favorise également la diffusion de ce gaz de stérilisation.

Au cours du processus de décontamination, comme le montre la figure 2, le plateau 5 vient reposer contre l'épaulement 11 et le rebord 23 vient reposer contre ce plateau 5, ce qui permet de restreindre, sinon d'empêcher, la diffusion de vapeurs de peroxyde d'hydrogène à l'intérieur du volume délimité par la plaque 20 et la boîte 3. La couche 6 repose contre les extrémités proximales des corps de seringue 2 et contribue à limiter la possibilité de pénétration de ces éventuelles vapeurs dans ces corps de seringue 2.

Dans la deuxième forme de réalisation montrée sur les figures 3 et 4, la plaque 20 est sous forme de grille, c'est-à-dire comprend une pluralité d'ouvertures entre les saillies 21 qu'elle comprend, et est dépourvue de jupe latérale 22 et de rebord 23.

Dans ce cas, l'emballage comprend une couche 8 flexible et non poreuse en remplacement de la couche 6, entre les corps de seringue 2 et la plaque 20. Cette couche 8 repose, lors du processus de stérilisation (cf. figure 3), dans les espaces délimités entre les saillies 21, de sorte que des passages subsistent vers l'intérieur des corps de seringue 2 pour permettre la diffusion du gaz de stérilisation dans ces corps 2. Au cours du processus de de décontamination (cf. figure 4), la plaque 20 porte contre les extrémités proximales des corps de seringue 2 et maintient ainsi la couche 8 plaquée contre ces extrémités proximales, ce qui permet de restreindre, sinon d'empêcher, la diffusion des vapeurs de peroxyde d'hydrogène à l'intérieur des corps de seringue 2.

Ainsi qu'il apparaît de ce qui précède, l'invention apporte une amélioration déterminante à la technique antérieure, en fournissant un emballage qui est efficace à l'égard d'éventuelles pénétrations d'un gaz de décontamination, par exemple de vapeurs de peroxyde d'hydrogène, lors du processus de décontamination sans amoindrissement significatif de l'aptitude de cet emballage à être stérilisé au moyen d'un gaz de stérilisation ; l'invention fournit également un procédé amélioré de stérilisation et de décontamination de cet emballage.

II va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées.

## Revendications

1. Emballage (1) destiné à être utilisé pour transporter des objets (2) stériles ou à stériliser, comprenant une boîte (3) destinée à recevoir les objets (2) stériles ou à stériliser et une feuille de couverture (4) en matériau sélectivement étanche, fixée sur la boîte (3) de manière à sceller cette dernière de façon étanche ;
cet emballage (1) comprenant au moins une couche (6, 8) d'un matériau formant un écran au moins partiel vis à vis d'un gaz de décontamination, par exemple vis-à-vis de vapeurs de peroxyde d'hydrogène, et/ou à même d'absorber un gaz de décontamination, par exemple des vapeurs de peroxyde d'hydrogène, cette couche (6, 8) ayant une forme et des dimensions telles qu'elle puisse être placée dans la boîte (3) le long de la feuille de couverture (4) et qu'elle s'étende, dans cette position, au-dessus des objets (2) contenus dans l'emballage (1), la ou lesdites couches (6, 8), ou les objets (2) étant mobiles entre une position de diffusion, permettant une diffusion non restreinte du gaz de stérilisation sur, entre et éventuellement dans, les objets (2), et une position de non diffusion, permettant une diffusion restreinte, voire empêchée, du gaz de décontamination, par exemple des vapeurs de peroxyde d'hydrogène, sur, entre et éventuellement dans, ces mêmes objets (2), et **caractérisé en ce qu'**il comprend en outre une plaque ou une grille (20) pourvue de saillies (21), conformée pour, dans ladite position de diffusion, permettre la diffusion non restreinte du gaz de stérilisation sur, entre et éventuellement dans, les objets (2), et, dans ladite position de non diffusion, restreindre ou interdire la diffusion du gaz de décontamination, par exemple des vapeurs de peroxyde d'hydrogène, sur, entre et éventuellement dans, ces mêmes objets (2).

2. Emballage (1) selon la revendication 1, **caractérisé en ce que** ladite couche ou au moins une desdites couches est rapportée sur la feuille de couverture notamment par collage ou soudure, et **en ce que** cette ou ces couches sont dimensionnées de manière à délimiter sur la feuille de couverture une zone périphérique de fixation de cette feuille de couverture à la boîte.

3. Emballage (1) selon la revendication 1, **caractérisé en ce que** ladite couche (6, 8) ou au moins une desdites couches (6, 8) sont disposées sur les objets (2) placés à l'intérieur de la boîte (3), préalablement au scellage de la feuille de couverture (4), ou sur des appuis prévus à cet effet, ou sur une pièce de positionnement des objets (2), placée dans cette boîte (3).

4. Emballage (1) selon la revendication 1, **caractérisé en ce qu'**il comprend au moins une desdites couches rapportée sur la feuille de couverture et au moins une autre desdites couches disposée à l'intérieur de la boîte.

5. Emballage (1) selon l'une des revendications 1 à 4, **caractérisé en ce qu'**il comprend plusieurs couches de matériau pour former ledit écran.

6. Emballage (1) selon la revendication 5, **caractérisé en ce que** les couches de matériau formant ledit écran sont identiques d'une couche à l'autre.

7. Emballage (1) selon la revendication 5, **caractérisé en ce que** les couches de matériau formant ledit écran sont différentes d'une couche à l'autre.

8. Emballage (1) selon l'une des revendications 1 à 7, **caractérisé en ce que** la ou les couches (6, 8) à même de former ledit écran sont conformées pour délimiter, lorsqu'elles sont en place dans la boîte (3), un ou plusieurs interstices ou ouvertures latérales ou périphériques entre leurs bords et les parois de cette boîte (3).

9. Emballage (1) selon la revendication 8, **caractérisé en ce que** la ou lesdites couches (6, 8) présentent des dimensions inférieures à celles de la boîte (3), de sorte qu'elles délimitent un ou plusieurs interstices entre leurs bords et les parois de cette boîte (3).

10. Emballage selon la revendication 8 ou la revendication 9, **caractérisé en ce que** la ou lesdites couches comprennent, au niveau de leurs bords, des encoches et/ou des découpes en forme de crénaux; de sorte qu'elles ménagent des ouvertures entre leurs bords et les parois de la boîte (3).

11. Emballage (1) selon l'une quelconque des revendications précédentes, **caractérisé en ce qu'**il est conformé de telle sorte que la ou lesdites couches (6, 8) et/ou les objets (2) passent de la position de diffusion à la position de non diffusion par gravité selon que l'emballage (1) est placé dans une première position, correspondant à la position de diffusion, ou qu'il est placé dans une deuxième position, renversée par rapport à ladite première position, correspondant à la position de non diffusion.

12. Emballage (1) selon l'une des revendications 1 à 11, **caractérisé en ce qu'**il comprend deux couches du matériau commercialisé sous la référence "TYVEK® 1073 B" par la société Du Pont de Nemours.

13. Emballage (1) selon l'une des revendications 1 à 11, **caractérisé en ce qu'**il comprend deux couches de papier grade médical, notamment en matériau commercialisé sous les dénominations "STERISHEET®", "PROPYPEL®", "ETHYPEL®", "ARPEEL®", "TALTER®" ou "TRANSPEL®" par la société Arjo Wiggins.

14. Emballage (1) selon l'une des revendications 1 à 11, **caractérisé en ce qu'**il comprend deux couches dont une est en matériau commercialisé sous la référence "TYVEK® 1073 B" par la société Du Pont de Nemours et l'autre est en papier grade médical, notamment en matériau commercialisé sous les dénominations "STERISHEET®", "PROPYPEL®", "ETHYPEL®", "ARPEEL®", "TALTER®" ou "TRANSPEL®" par la société Arjo Wiggins.

15. Emballage (1) selon l'une des revendications 1 à 11, **caractérisé en ce qu'**il comprend deux couches dont une est en matériau commercialisé sous la référence "TYVEK® 1073 B" par la société Du Pont de Nemours et l'autre est en matériau commercialisé sous la référence "TYVEK® 2FS" par la société Du Pont de Nemours.

16. Emballage (1) selon l'une des revendications 1 à 11, **caractérisé en ce qu'**il comprend deux couches dont une est en matériau commercialisé sous la référence "TYVEK® 2FS" par la société Du Pont de Nemours et l'autre est en papier grade médical, notamment en matériau commercialisé sous les dénominations "STERISHEET®", "PROPYPEL®", "ETHYPEL®", "ARPEEL®", "TALTER®" ou "TRANSPEL®" par la société Arjo Wiggins.

17. Emballage (1) selon la revendication 15 ou la revendication 16, **caractérisé en ce que** le côté lisse du matériau commercialisé sous la référence "TYVEK® 2FS" est placé en contact avec les objets contenus dans l'emballage.

18. Procédé de fabrication de l'emballage (1) selon l'une des revendications 1 à 17, comprenant les étapes consistant à :
- utiliser au moins un matériau à même de former un écran au moins partiel vis à vis d'un gaz de décontamination, par exemple vis-à-vis de vapeurs de peroxyde d'hydrogène, et/ou à même d'absorber un gaz de décontamination, par exemple des vapeurs de peroxyde d'hydrogène,
- aménager au moins une couche (6, 8) de ce matériau, en choisissant la forme et les dimensions de cette couche (6, 8) de telle sorte que celle-ci puisse être placée dans la boîte (3) le long de la feuille de couverture (4) et qu'elle s'étende, dans cette position, au-dessus des objets (2) contenus dans l'emballage (1), et **caractérisé en ce qu**'il comprend également l'étape consistant à :
- aménager une plaque ou une grille (20) pourvue de saillies (21), conformée pour, dans une position de diffusion, permettre la diffusion non restreinte du gaz de stérilisation sur, entre et éventuellement dans, les objets (2), et, dans une position de non diffusion, restreindre ou interdire la diffusion du gaz de décontamination, par exemple des vapeurs de peroxyde d'hydrogène, sur, entre et éventuellement dans, ces mêmes objets (2).

19. Procédé selon la revendication 18, **caractérisé en ce qu'**il comprend les étapes consistant à :
- dimensionner ladite couche ou au moins une desdites couches de telle sorte que, lorsque cette couche est rapportée sur la feuille de couverture (4), elle délimite sur cette feuille de couverture (4) une zone périphérique de fixation de cette feuille de couverture (4) à la boîte (3) ; et
- rapporter ladite couche ou au moins une desdites couches sur la feuille de couverture (4), notamment par collage ou soudure.

20. Procédé selon la revendication 18 ou la revendication 19, **caractérisé en ce qu'**il comprend l'étape consistant à disposer ladite couche ou au moins une desdites couches (6, 8) sur les objets (2) placés à l'intérieur de la boîte (3), préalablement au scellage de la feuille de couverture (4), ou sur des appuis prévus à cet effet, ou sur une pièce de positionnement des objets (2), placée dans cette boîte (3).

21. Procédé selon l'une des revendications 18 à 19, **caractérisé en ce qu'**il comprend les étapes consistant à :
- dimensionner au moins une desdites couches de telle sorte que, lorsque cette couche est rapportée sur la feuille de couverture (4), elle délimite sur cette feuille de couverture (4) une zone périphérique de fixation de cette feuille de couverture (4) à la boîte (3) ;
- rapporter cette ou ces couches ou au moins une desdites couches sur la feuille de couverture (4), notamment par collage ou soudure ; et
- disposer au moins une autre desdites couches sur les objets (2) placés à l'intérieur de la boîte (3), préalablement au scellage de la feuille de couverture (4), ou sur des appuis prévus à cet effet, ou sur une pièce de positionnement des objets (2), placée dans cette boîte (3).

22. Procédé de stérilisation et de décontamination utilisant l'emballage (1) selon la revendication 1 **caractérisé en ce qu'**il comprend les étapes consistant à :
- placer l'emballage (1) en position de diffusion au cours du processus de stérilisation ; et
- placer l'emballage (1) en position de non diffusion au cours du processus de décontamination.

23. Procédé de stérilisation et de décontamination utilisant l'emballage (1) selon la revendication 11 **caractérisé en ce qu'**il comprend les étapes consistant à :
- placer l'emballage (1) dans une première position au cours du processus de stérilisation, dans laquelle la ou lesdites couches (6, 8) et les objets (2) sont en position de diffusion, de sorte que cette ou ces couches (6, 8) ne restreignent que modérement, voire pas du tout, la diffusion du gaz de stérilisation sur, entre et éventuellement dans, les objets (2) ; et
- placer l'emballage (1) dans une deuxième position au cours du processus de décontamination, dans laquelle la ou lesdites couches (6, 8) et les objets (2) sont en position de non diffusion, de sorte que cette ou ces couches (6, 8) restreignent, voire interdisent, la diffusion du gaz de décontamination, par exemple des vapeurs de peroxyde d'hydrogène, sur, entre et éventuellement dans, ces objets (2).

24. Utilisation de l'emballage (1) selon l'une des revendications 1 à 17 dans un procédé de décontamination de cet emballage par un gaz de décontamination, par exemple par des vapeurs de peroxyde d'hydrogène.

25. Utilisation de l'emballage (1) selon l'une des revendications 1 à 17 pour transporter des composants de seringues, en particulier des corps de seringue (2) destinés à être ultérieurement remplis par un produit actif ou un médicament.

## Patentansprüche

1. Verpackung (1), die dazu bestimmt ist, zum Transportieren von sterilen oder zu sterilisierenden Gegenständen (2) verwendet zu werden, mit einer Schachtel (3), die dazu bestimmt ist, die sterilen oder zu sterilisierenden Gegenstände (2) aufzunehmen, und einer Abdeckfolie (4) aus einem selektiv dichten Material, die auf der Schachtel (3) derart befestigt ist, dass sie letztere dicht verschließt;
wobei die Verpackung (1) zumindest eine Lage (6, 8) aus einem Material aufweist, das eine zumindest teilweise Abschirmung gegenüber einem Dekontaminiergas, bspw. gegenüber Wasserstoffperoxiddämpfen, bildet, und/oder in der Lage ist, ein Dekontaminiergas, bspw. Wasserstoffperoxiddämpfe, zu absorbieren, wobei die Lage (6, 8) eine solche Form und solche Abmessungen aufweist, dass sie in der Schachtel (3) entlang der Abdeckfolie (4) angeordnet werden kann und sich in dieser Position oberhalb der in der Verpackung (1) enthaltenen Gegenstände (2) erstreckt, wobei die Lage oder die Lagen (6, 8) oder die Gegenstände (2) zwischen einer Diffusionsstellung, die eine uneingeschränkte Diffusion des Sterilisiergases an, zwischen und ggf. in die Gegenstände (2) ermöglicht, und einer Nicht-Diffusionsstellung beweglich ist/sind, die eine eingeschränkte Diffusion des Dekontaminiergases, bspw. von Wasserstoffperoxiddämpfen, an, zwischen und ggf. in die Gegenstände (2) ermöglicht oder eine solche Diffusion sogar verhindert, **dadurch gekennzeichnet, dass** sie außerdem eine Platte oder ein Gitter (20) aufweist, die bzw. das mit Vorsprüngen (21) versehen ist und dazu ausgelegt ist, in der Diffusionsstellung die uneingeschränkte Diffusion des Sterilisiergases an, zwischen und ggf. in die Gegenstände (2) zu ermöglichen und in der Nicht-Diffusionsstellung die Diffusion des Dekontaminiergases, bspw. von Wasserstoffperoxiddämpfen, an, zwischen und ggf. in dieselben Gegenstände (2) einzuschränken oder zu verhindern.

2. Verpackung (1) nach Anspruch 1, **dadurch gekennzeichnet, dass** die Lage oder die zumindest eine der Lagen an der Abdeckfolie insbesondere durch Klebung oder Schweißverbindung angebracht ist, und dass die Lage oder die Lagen derart dimensioniert ist/sind, dass sie an der Abdeckfolie eine umfängliche Zone der Befestigung der Abdeckfolie an der Schachtel abgrenzen.

3. Verpackung (1) nach Anspruch 1, **dadurch gekennzeichnet, dass** die Lage (6, 8) oder die zumindest eine der Lagen (6, 8) vor dem Verschließen der Abdeckfolie (4) auf den im Inneren der Schachtel (3) angeordneten Gegenständen (2) oder auf zu diesem Zweck vorgesehenen Auflagen oder auf einem Positionierstück für die Gegenstände (2), das in der Schachtel (3) angeordnet ist, angeordnet wird.

4. Verpackung (1) nach Anspruch 1, **dadurch gekennzeichnet, dass** sie zumindest eine an der Abdeckfolie angebrachte Lage und zumindest eine weitere im Inneren der Schachtel angeordnete Lage aufweist.

5. Verpackung (1) nach einem der Ansprüche 1 bis 4, **dadurch gekennzeichnet, dass** sie mehrere Materiallagen aufweist, um die Abschirmung zu bilden.

6. Verpackung (1) nach Anspruch 5, **dadurch gekennzeichnet, dass** die Materiallagen, die die Abschirmung bilden, von Lage zu Lage identisch sind.

7. Verpackung (1) nach Anspruch 5, **dadurch gekennzeichnet, dass** die Materiallagen, die die Abschirmung bilden, von einer Lage zur anderen unterschiedlich sind.

8. Verpackung (1) nach einem der Ansprüche 1 bis 7, **dadurch gekennzeichnet, dass** die Lage oder die Lagen (6, 8), die die Abschirmung bilden kann/können, dazu ausgelegt ist/sind, einen oder mehrere seitliche oder umfängliche Zwischenräume oder Öffnungen zwischen ihren Rändern und den Wänden der Schachtel (3) abzugrenzen.

9. Verpackung (1) nach Anspruch 8, **dadurch gekennzeichnet, dass** die Lage oder die Lagen (6, 8) geringere Abmessungen aufweist/aufweisen als diejenigen der Schachtel (3), derart, dass sie einen oder mehrere Zwischenräume zwischen ihren Rändern und den Wänden der Schachtel (3) abgrenzt/abgrenzen.

10. Verpackung nach Anspruch 8 oder Anspruch 9, **dadurch gekennzeichnet, dass** die Lage oder die Lagen im Bereich ihrer Ränder kerbenförmige Aussparungen und/oder Ausschnitte aufweist/aufweisen, derart, dass sie Öffnungen zwischen ihren Rändern und den Wänden der Schachtel (3) aussparen.

11. Verpackung (1) nach einem der vorhergehenden Ansprüche, **dadurch gekennzeichnet, dass** sie derart ausgelegt ist, dass die Lage oder die Lagen (6, 8) und/oder die Gegenstände (2) von der Diffusionsstellung in die Nicht-Diffusionsstellung durch Schwerkraft übergeht/übergehen, je nach dem ob die Verpackung (1) in einer ersten Stellung, die der Diffusionsstellung entspricht, oder in einer zweiten Stellung angeordnet wird, die bezüglich der ersten Stellung umgekehrt ist und der Nicht-Diffusionsstellung entspricht.

12. Verpackung (1) nach einem der Ansprüche 1 bis 11, **dadurch gekennzeichnet, dass** sie zwei Lagen aus dem unter der Referenz "TYVEK® 1073 B" von der Firma Du Pont de Nemours vertriebenen Material aufweist.

13. Verpackung (1) nach einem der Ansprüche 1 bis 11, **dadurch gekennzeichnet, dass** sie zwei Lagen aus medizinischem Papier, insbesondere aus einem unter den Bezeichnungen "STERISHEET®", "PROPYPEL®", "ETHYPEL®", "ARPEEL®", "TALTER®" oder "TRANSPEL®" von der Firma Arjo Wiggins vertriebenen Material ist.

14. Verpackung (1) nach einem der Ansprüche 1 bis 11, **dadurch gekennzeichnet, dass** sie zwei Lagen aufweist, von denen eine aus dem unter der Referenz "TYVEK® 1073 B" von der Firma Du Pont de Nemours vertriebenen Material ist, und die andere aus medizinischem Papier, insbesondere aus einem unter den Kennzeichnungen "STERISHEET®", "PROPYPEL®", "ETHYPEL®", "ARPEEL®", "TALTER®" oder "TRANSPEL®" von der Firma Arjo Wiggins vertriebenen Material ist.

15. Verpackung (1) nach einem der Ansprüche 1 bis 11, **dadurch gekennzeichnet, dass** sie zwei Lagen aufweist, von denen eine aus dem unter der Referenz "TYVEK® 1073 B" von der Firma Du Pont de Nemours vertriebenen Material ist, und die andere aus dem unter der Referenz "TYVEK® 2FS" von der Firma Du Pont de Nemours vertriebenen Material ist.

16. Verpackung (1) nach einem der Ansprüche 1 bis 11, **dadurch gekennzeichnet, dass** sie zwei Lagen aufweist, von denen eine aus dem unter der Referenz "TYVEK® 2FS" von der Firma Du Pont de Nemours vertriebenen Material ist, und die andere aus einem medizinischen Papier, insbesondere aus einem unter den Kennzeichnungen "STERISHEET®", "PROPYPEL®", "ETHYPEL®", "ARPEEL®", "TALTER®" oder "TRANSPEL®" von der Firma Arjo Wiggins vertriebenen Material ist.

17. Verpackung (1) nach Anspruch 15 oder Anspruch 16, **dadurch gekennzeichnet, dass** die glatte Seite des unter der Referenz "TYVEK® 2FS" vertriebenen Materials in Berührung mit den in der Verpackung enthaltenen Gegenständen angeordnet ist.

18. Verfahren zum Herstellen der Verpackung (1) nach einem der Ansprüche 1 bis 17, mit den Schritten:
- Verwenden zumindest eines Materials, das in der Lage ist, eine zumindest teilweise Abschirmung gegenüber einem Dekontaminiergas, bspw. gegenüber Wasserstoffperoxiddämpfen, zu bilden, und/oder das in der Lage ist, ein Dekontaminiergas, bspw. Wasserstoffperoxiddämpfe, zu absorbieren,
- Herrichten zumindest einer Lage (6, 8) aus diesem Material, indem die Form und die Abmessungen dieser Lage (6, 8) derart gewählt werden, dass sie in der Schachtel (3) entlang der Abdeckfolie (4) angeordnet werden kann und sich in dieser Stellung oberhalb der in der Verpackung (1) enthaltenen Gegenstände (2) erstreckt, **dadurch gekennzeichnet, dass** es außerdem den Schritt umfasst:
- Herrichten einer Platte oder eines Gitters (20), die/das mit Vorsprüngen (21) versehen ist, und die/das dazu ausgelegt ist, in einer Diffusionsstellung die uneingeschränkte Diffusion des Sterilisiergases an, zwischen und ggf. in die Gegenstände (2) zu ermöglichen, und in einer Nicht-Diffusionsstellung die Diffusion des Dekontaminiergases, bspw. von Wasserstoffperoxiddämpfen, an, zwischen und ggf. in dieselben Gegenstände (2) einzuschränken oder zu verhindern.

19. Verfahren nach Anspruch 18, **dadurch gekennzeichnet, dass** es folgende Schritte umfasst:
- Abmessen der Lage oder zumindest einer der Lagen derart, dass diese Lage, wenn sie an der Abdeckfolie (4) angebracht ist, an dieser Abdeckfolie (4) eine umfängliche Zone der Befestigung der Abdeckfolie (4) an der Schachtel (3) abgrenzt; und
- Anbringen der Lage oder der zumindest einen der Lagen an der Abdeckfolie (4) insbesondere durch Klebung oder Schweißverbindung.

20. Verfahren nach Anspruch 18 oder Anspruch 19, **dadurch gekennzeichnet, dass** es den Schritt umfasst, die Lage oder die zumindest eine der Lagen (6, 8) vor dem Verschließen der Abdeckfolie (4) auf den im Inneren der Schachtel (3) angeordneten Gegenständen oder auf zu diesem Zweck vorgesehenen Auflagen oder auf einem Positionierstück für die Gegenstände (2), das in der Schachtel (3) angeordnet ist, anzuordnen.

21. Verfahren nach einem der Ansprüche 18 bis 19, **dadurch gekennzeichnet, dass** es die folgenden Schritte umfasst:
- Abmessen der Lage oder zumindest einer der Lagen derart, dass diese Lage, wenn sie an der Abdeckfolie (4) angebracht ist, an dieser Abdeckfolie (4) eine umfängliche Zone der Befestigung der Abdeckfolie (4) an der Schachtel (3) abgrenzt; und
- Anbringen der Lage oder der zumindest einen der Lagen an der Abdeckfolie (4) insbesondere durch Klebung oder Schweißverbindung; und
- Anordnen zumindest einer weiteren der Lagen vor dem Verschließen der Abdeckfolie (4) auf den Gegenständen (2), die im Inneren der Schachtel (3) angeordnet sind, oder auf zu diesem Zweck vorgesehenen Auflagen, oder auf einem Positionierstück für die Gegenstände (2), das in der Schachtel (3) angeordnet ist.

22. Verfahren zum Sterilisieren und Dekontaminieren, bei dem die Verpackung (1) nach Anspruch 1 verwendet wird, **dadurch gekennzeichnet, dass** es die folgenden Schritte umfasst:
- Anordnen der Verpackung (1) in der Diffusionsstellung während des Sterilisiervorgangs; und
- Anordnen der Verpackung (1) in der Nicht-Diffusionsstellung während des Dekontaminiervorgangs.

23. Verfahren zum Sterilisieren und Dekontaminieren, bei dem die Verpackung (1) nach Anspruch 11 verwendet wird, **dadurch gekennzeichnet, dass** es die folgenden Schritte umfasst:
- Anordnen der Verpackung (1) in einer ersten Stellung während des Sterilisiervorgangs, in der die Lage oder die Lagen (6, 8) und die Gegenstände (2) in der Diffusionsstellung sind, derart, dass diese Lage oder diese Lagen (6, 8) die Diffusion des Sterilisiergases an, zwischen und ggf. in die Gegenstände (2) nur mäßig oder gar nicht einschränkt; und
- Anordnen der Verpackung (1) in einer zweiten Stellung während des Dekontaminiervorgangs, in der die Lage oder die Lagen (6, 8) und die Gegenstände (2) in einer Nicht-Diffusionsstellung sind, derart, dass diese Lage oder diese Lagen (6, 8) die Diffusion des Dekontaminiergases , bspw. von Wasserstoffperoxiddämpfen, an, zwischen und ggf. in die Gegenstände (2) einschränkt/einschränken oder sogar verhindert/verhindern.

24. Verwendung der Verpackung (1) nach einem der Ansprüche 1 bis 17 in einem Verfahren zum Dekontaminieren dieser Verpackung durch ein Dekontaminiergas, bspw. durch Wasserstoffperoxiddämpfe.

25. Verwendung der Verpackung (1) nach einem der Ansprüche 1 bis 17 zum Transportieren von Spritzenkomponenten, insbesondere von Spritzenkörpern (2), die dazu bestimmt sind, später mit einem wirksamen Produkt oder einem Medikament gefüllt zu werden.

## Claims

1. A package (1) intended to be used to transport objects (2) which are sterile or to be sterilized, comprising a box (3) intended to accommodate objects (2) which are sterile or to be sterilized and a covering sheet (4) made of a selectively leaktight material, fastened onto the box (3) so as to seal the latter in a leaktight manner;
package (1) which comprises at least one layer (6, 8) of a material forming a screen which is at least partial with respect to a decontamination gas, for example, with respect to hydrogen peroxide vapor, and/or able to absorb a decontamination gas, for example, hydrogen peroxide vapor, this layer (6, 8) having a shape and dimensions such that it can be placed in the box (3) along the covering sheet (4) and that it lies, in this position, above the objects (2) contained in the package (1), said layer or layers (6, 8), or the objects (2) being mobile between a diffusion position, allowing unrestricted diffusion of the sterilization gas over, between and possibly within the objects (2), and a nondiffusion position, enabling restricted, or even prevented diffusion of the decontamination gas, for example, the hydrogen peroxide vapor over, between and possibly within these same objects (2), and **characterized in that** it further comprises a plate or a grid (20) provided with projections (21), shaped in order, in said diffusion position, to allow unrestricted diffusion of the sterilization gas over, between and possibly within the objects (2), and, in said nondiffusion position, to restrict or prevent diffusion of the decontamination gas, for example of the hydrogen peroxide vapor over, between and possibly within these same objects (2).

2. The package (1) as claimed in claim 1, **characterized in that** said layer or at least one of said layers is attached to the covering sheet, especially by adhesive bonding or welding, and **in that** this layer or these layers are dimensioned so as to define, on the covering sheet, a peripheral region for fastening this covering sheet to the box.

3. The package (1) as claimed in claim 1, **characterized in that** said layer (6, 8) or at least one of said layers (6, 8) are arranged on the objects (2) placed inside the box (3), prior to sealing the covering sheet (4), or on supports provided to this end, or on a part for positioning objects (2), placed in this box (3).

4. The package (1) as claimed in claim 1, which comprises at least one of said layers attached to the covering sheet and at least one other of said layers arranged inside the box.

5. The package (1) as claimed in one of claims 1 to 4, which comprises several layers of material in order to form said screen.

6. The package (1) as claimed in claim 5, **characterized in that** the layers of material forming said screen are identical from one layer to the other.

7. The package (1) as claimed in claim 5, **characterized in that** the layers of material forming said screen are different from one layer to the other.

8. The package (1) as claimed in one of claims 1 to 7, **characterized in that** the layer or layers (6, 8) able to form said screen are shaped in order to define, when they are in place in the box (3), one or more lateral or peripheral openings or interstices between their edges and the walls of this box (3).

9. The package (1) as claimed in claim 8, **characterized in that** said layer or layers (6, 8) have smaller dimensions than those of the box (3), such that they define one or more interstices between their edges and the walls of this box (3).

10. The package as claimed in claim 8 or claim 9, **characterized in that** said layer or layers comprise, at their edges, notches and/or cutouts in the form of pegs; such that they make openings between their edges and the walls of the box (3).

11. The package as claimed in any one of the preceding claims, **characterized in that** it is shaped such that the layer or layers (6, 8) and/or the objects (2) go from the diffusion position to the nondiffusion position by gravity depending on whether the package (1) is placed in a first position corresponding to the diffusion position, or whether it is placed in a second position, reversed with respect to said first position, corresponding to the nondiffusion position.

12. The package (1) as claimed in one of claims 1 to 11, which comprises two layers of the material marketed under the reference "TYVEK® 1073 B" by DuPont de Nemours.

13. The package (1) as claimed in one of claims 1 to 11, which comprises two layers of medical grade paper, especially made of a material marketed under the name "STERISHEET®", "PROPYPEL®", "ETHYPEL®", "ARPEEL®", "TALTER®" or "TRANSPEL®" by Arjo Wiggins.

14. The package (1) as claimed in one of claims 1 to 11, which comprises two layers, one of which is made of a material marketed under the reference "TYVEK® 1073 B" by DuPont de Nemours and the other is made of a medical grade paper, especially made of a material marketed under the names "STERISHEET®", "PROPYPEL®", "ETHYPEL®", "ARPEEL®", "TALTER®" or "TRANSPEL®" by Arjo Wiggins.

15. The package (1) as claimed in one of claims 1 to 11, which comprises two layers, one of which is made of a material marketed under the reference "TYVEK® 1073 B" by DuPont de Nemours and the other is made of a material marketed under the reference "TYVEK® 2FS" by DuPont de Nemours.

16. The package (1) as claimed in one of claims 1 to 11, which comprises two layers, one of which is made of a material marketed under the reference "TYVEK® 2FS" by DuPont de Nemours and the other is made of a medical grade paper, especially made of a material marketed under the names "STERISHEET®", "PROPYPEL®", "ETHYPEL®", "ARPEEL®", "TALTER®" or "TRANSPEL®" by Arjo Wiggins.

17. The package (1) as claimed in claim 15 or claim 16, **characterized in that** the smooth side of the material marketed under the reference "TYVEK® 2FS" is placed in contact with the objects contained in the package.

18. A process of fabricating the package (1) as claimed in one of claims 1 to 17, which comprises the steps consisting in:
- using at least one material capable of forming a screen which is at least partial with respect to a decontamination gas, for example with respect to hydrogen peroxide vapor, and/or capable of absorbing a decontamination gas, for example hydrogen peroxide vapor,
- making at least one layer (6, 8) of this material, while choosing the shape and the dimensions of this layer (6, 8) such that the latter can be placed in the box (3) along the covering sheet (4) and that it lies, in this position, above the objects (2) contained in the package (1), and **characterized in that** it further comprises the step consisting in :
- making a plate or a grid (20) provided with projections (21) shaped in order, in a diffusion position, to allow unrestricted diffusion of the sterilization gas over, between and possibly within the objects (2), and, in a nondiffusion position, to restrict or prevent diffusion of the decontamination gas, for example of the hydrogen peroxide vapor over, between and possibly within these same objects (2).

19. The process as claimed in claim 18, which comprises the steps consisting in:
- dimensioning said layer or at least one of said layers such that, when this layer is attached to the covering sheet (4), it defines on this covering sheet (4) a peripheral region for fastening this covering sheet (4) to the box (3); and
- attaching said layer or at least one of said layers to the covering sheet (4), especially by adhesive bonding or welding.

20. The process as claimed in claim 18 or claim 19, which comprises the step consisting in arranging said layer or at least one of said layers (6, 8) on the objects (2) placed inside the box (3), prior to sealing the covering sheet (4), or on supports provided to this end, or on a part for positioning the objects (2), placed in this box (3).

21. The process as claimed in one of claims 18 to 19, which comprises the steps consisting in:
- dimensioning at least one of said layers such that, when this layer is attached to the covering sheet (4), it defines on this covering sheet (4) a peripheral region for fastening this covering sheet (4) to the box (3);
- attaching this layer or these layers or at least one of said layers to the covering sheet (4), especially by adhesive bonding or welding; and
- arranging at least one other of said layers on the objects (2) placed inside the box (3), prior to sealing the covering sheet (4), or on supports provided to this end, or on a part for positioning the objects (2), placed in this box (3).

22. A sterilizing and decontamination process using the package (1) as claimed in claim 1, which comprises the steps consisting in:
- placing the package (1) in the diffusion position during the sterilization process; and
- placing the package (1) in the nondiffusion position during the decontamination process.

23. The sterilization and decontamination process using the package (1) as claimed in claim 11, which comprises the steps consisting in:
- placing the package (1) in a first position during the sterilization process, in which said layer or layers (6, 8) and the objects (2) are in the diffusion position, such that this layer or these layers (6, 8)restrict only moderately, or even not at all, the diffusion of the sterilization gas over, between and possibly within the objects (2); and
- placing the package (1) in a second position during the decontamination process, in which said layer or layers (6, 8) and the objects (2) are in the nondiffusion position, such that this layer or these layers (6, 8) restrict, or even prevent, the diffusion of the decontamination gas, for example, of the hydrogen peroxide vapor over, between and possibly within these objects (2).

24. The use of the package (1) as claimed in one of claims 1 to 17 in a process for decontaminating this package by a decontamination gas, for example, by hydrogen peroxide vapor.

25. The use of the package (1) as claimed in one of claims 1 to 17 in order to transport syringe components, in particular syringe bodies (2) intended to be filled subsequently by an active product or a medication.
